# FASCICULE DE BREVET EUROPEEN

(11) **EP 1 331 917 B1**
(45) Date de publication et mention de la délivrance du brevet: **25.10.2006**
(21) Numéro de dépôt: 01982554.6
(22) Date de dépôt: 25.10.2001
(51) Int. Cl.: A61Q 5/00, A61K 8/04, A61K 8/86

(54) **COMPOSITION COSMETIQUE CAPILLAIRE SOUS FORME DE MOUSSE COMPRENANT AU MOINS UN POLYMERE EPAISSISSANT A SQUELETTE AMINOPLASTE-ETHER**
SCHAUMFÖRMIGE HAARKOSMETISCHE ZUBEREITUNG MIT EINEM AMINOPLASTETHER ALS VERDICKUNGSMITTEL
FOAM-LIKE COSMETIC HAIR COMPOSITION COMPRISING AT LEAST A AMINOPLAST-ETHER SKELETON THICKENING POLYMER

(30) Priorité: 27.10.2000 FR 0013867
(43) Date de publication de la demande: 06.08.2003
(60) Demande divisionnaire: 05292223.4
(73) Titulaire: L'ORÉAL, 75008 Paris (FR)
(72) Inventeur: DUPUIS, Christine, F-75018 Paris (FR); MINOU, Patrick, F-95150 Taverny (FR)
(74) Mandataire: Bourdeau, Françoise
(86) Numéro de dépôt international: PCT/FR2001/003317
(87) Numéro de publication internationale: WO 2002/034217

(56) Documents cités:
- US-A- 5 627 232
- US-A- 5 914 373

## Description

L'invention a pour objet une composition cosmétique capillaire se présentant sous forme de mousse et comprenant au moins un polymère épaississant à squelette aminoplaste éther.

Elle vise également son utilisation pour la fabrication de formulations capillaires telles que des produits de soin, des produits de conditionnement ou des produits pour la fixation et/ou le maintien des cheveux.

Les compositions cosmétiques capillaires contiennent, en général, au moins un polymère de préférence anionique, non ionique ou amphotère. Ils peuvent, par exemple, apporter des propriétés de fixation des cheveux.

Les formulations les plus couramment utilisées se présentent le plus souvent sous forme de mousse aérosol, de spray aérosol, de spray flacon pompe ou bien de gel.

Les mousses permettent généralement d'obtenir sur les cheveux une bonne répartition des compositions cosmétiques et elles sont, en outre, d'une utilisation aisée. Les polymères utilisés dans ces produits étant généralement non moussants ou faiblement moussants, il est nécessaire d'ajouter un agent moussant et/ou un agent améliorant la qualité de la mousse.

Les agents moussants et/ou les agents améliorant la qualité de la mousse habituellement utilisés sont, par exemple, des agents tensioactifs anioniques, non ioniques ou amphotères. Toutefois, ces agents tensioactifs provoquent parfois un remoussage sur cheveux mouillés, ce qui nuit à une réalisation rapide et soignée de la coiffure.

Par ailleurs, l'application de mousse sur les cheveux présente parfois l'inconvénient d'alourdir la coiffure. Enfin, la réalisation de mousses nécessite soit un dispositif aérosol, soit un dispositif pompe mousse.

En général, la réalisation de compositions capillaires sous forme de mousse est réalisée grâce à l'introduction de tensioactifs moussants ou de polymères moussants. Dans le premier cas, la composition donne souvent des résultats peu satisfaisant en terme de propriétés cosmétiques, dans le second, les polymères moussants risquent d'être incompatibles avec le reste de la formulation. En outre, il est nécessaire d'utiliser une quantité en polymère moussant qui est importante, ce qui nuit à la qualité des résultats cosmétiques. Ces inconvénients existent d'ailleurs surtout pour des compositions moussantes non rincées.

Dans le cas spécifique des pompes mousses, la recherche d'un compromis entre la qualité de la mousse et les qualités cosmétiques est particulièrement délicat.

Le problème posé par l'invention est de réaliser des compositions cosmétiques capables de mousser sans faire intervenir d'agent tensioactif et en utilisant une quantité restreinte d'agent moussant, pour obtenir une mousse de qualité satisfaisante, de même que de propriétés cosmétiques satisfaisantes.

De manière surprenante et inattendue, la Demanderesse a découvert, contre toute attente, qu'il était possible de réaliser des compositions cosmétiques capillaires se présentant sous forme de mousse et qui ne présentent pas les inconvénients exprimés ci-dessus, en choisissant, en tant que constituant de la composition cosmétique, un polymère particulier qui sera défini plus en détail par la suite.

L'invention a pour objet une composition cosmétique capillaire se présentant sous forme de mousse conditionnée en aérosol ou en flacon pompe sans propulseur et comprenant, dans un milieu cosmétiquement acceptable, au moins un polymère à squelette aminoplaste-éther.

Par « aminoplaste-éther », on entend au sens de la présente invention, tout produit issu de la condensation d'un aldéhyde avec une amine ou un amide.

Par « aminoplaste-éther », on entend aussi au sens de la présente invention, toute unité structurale formée d'un résidu aminoplaste et d'un résidu hydrocarboné divalent lié au résidu aminoplaste par une liaison éther.

Un autre objet de l'invention concerne un procédé cosmétique capillaire mettant en oeuvre cette composition.

Un autre objet de l'invention concerne plus particulièrement un procédé de fixation et/ou de maintien des cheveux mettant en oeuvre cette composition.

Encore un autre objet de l'invention concerne l'utilisation de cette composition pour la fabrication de produits capillaires, notamment ceux destinés à la fixation et/ou la mise en forme de la coiffure.

Encore un autre objet de la présente invention concerne l'utilisation d'un polymère à squelette aminoplaste-éther, comme agent moussant dans une composition cosmétique.

Au sens de la présente invention, on entend par « mousse », une structure microaérée de faible densité, bien connue de l'homme de l'art.

Les polymères à squelette aminoplaste-éther utilisés selon l'invention, sont choisis de préférence parmi ceux contenant au moins un motif de structure (I) suivante : 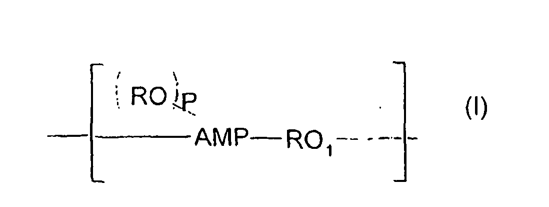 dans laquelle :
- AMP est un résidu aminoplaste avec unités alkylènes,
- R désigne un atome d'hydrogène, un radical alkyl C₁C₄ ou un radical acyle C₁C₄,
- RO₁ est un résidu alkylèneoxy divalent,
- p désigne un nombre entier positif,
le ou les groupements OR étant liés aux unités alkylènes du résidu AMP. De préférence, les polymères à squelette aminoplaste-éther sont choisis parmi ceux contenant au moins un motif de structure (II) suivante : 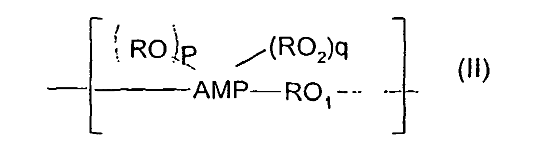 dans laquelle :
- AMP, R, RO₁ et p ont la même signification que précédemment,
- RO₂ est un groupe hydrophobe différent de RO lié à AMP via un hétéroatome et comprenant au moins deux atomes de carbone, et,
- q est un nombre entier positif.

Plus préférentiellement encore, les polymères de l'invention sont de formules (III) et (III)bis suivantes : 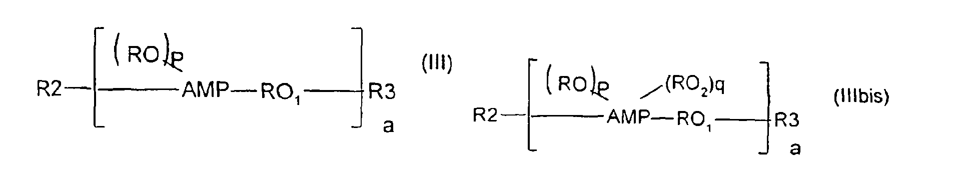 dans lesquelles:
AMP, R, RO₁, RO₂, p et q ont la même signification que précédemment, R2 ou R3, identiques ou différents représentent un groupe terminal pouvant désigner un atome d'hydrogène, un groupement RO₁H, un groupement RO₂H, un groupement AMP(OR)p ou tout groupement monofonctionnel tel que alkyle, cycloalkyle, aryle, aralkyle, alkylaryle, alkyloxyalkyle, aryloxyalkyle, cycloalkoxyalkyle,
a étant un nombre plus grand que 1 et de préférence plus grand que 2.

Les résidus aminoplastes porteurs de leurs groupements OR intégrés dans les polymères de l'invention peuvent être choisis de manière non limitative parmi les structures (IV) à (XV) suivantes : 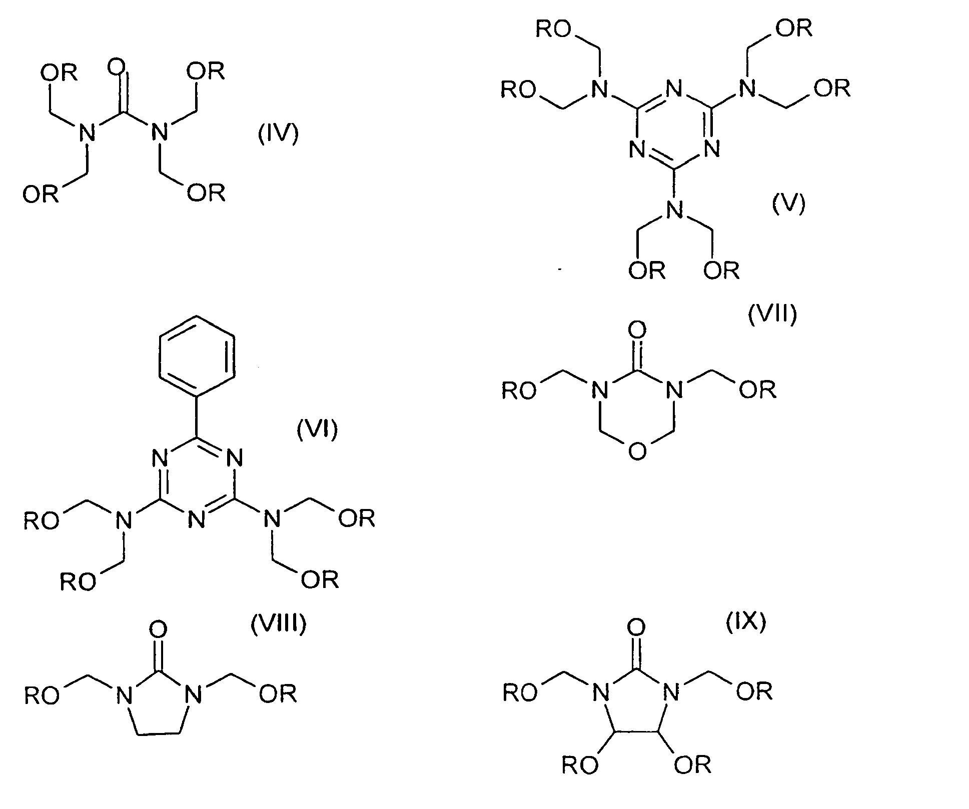 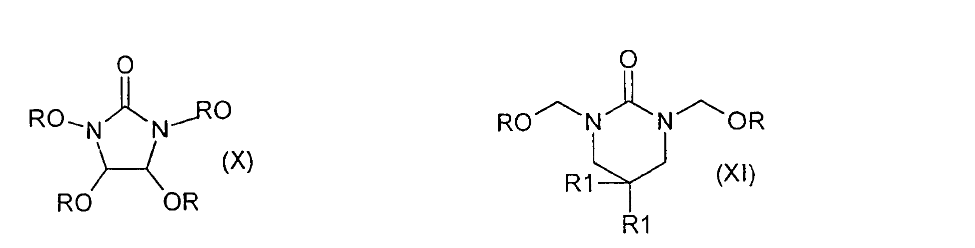 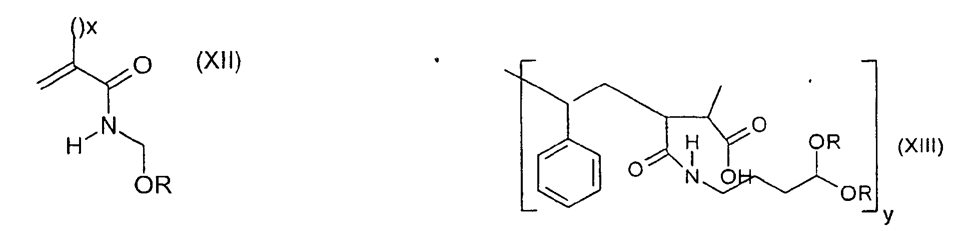 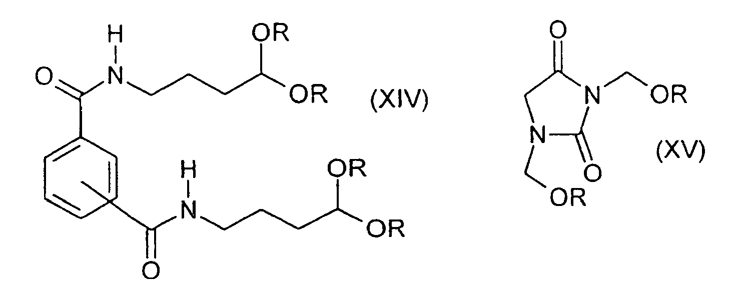 dans lesquelles :
R a la même signification que précédemment,
R1 désigne alkyle C₁C₄,
y est un nombre au moins égal à 2,
x désigne 0 ou 1.

De préférence, le ou les résidus aminoplastes porteurs de leurs groupements OR sont choisis parmi ceux de structure (XVI) suivante : 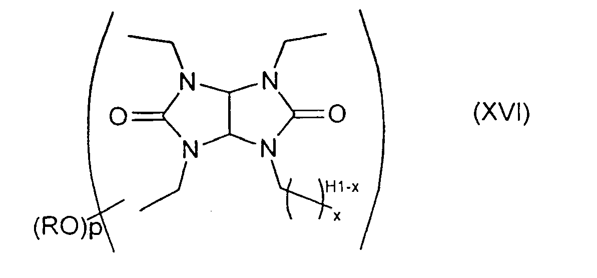 dans laquelle R, p , et x ont les mêmes significations que précédemment.

Les résidus alkylèneoxy divalents sont de préférence ceux correspondants aux diols de formule générale (XVII) suivante :

HO-(ZO)y-(Z1(Z20)w)t-(Z'O)y'-Z3OH (XVII),

y et y' étant des nombres allant de 0 à 1000,
t et w étant des nombres allant de 0 à 10,
Z, Z', Z2 et Z3 sont des radicaux alkylène en C₂C₄ et de préférence des radicaux -CH2-CH(Z4)- et -CH2-CH(Z4)-CH2-,
Z1 étant un radical linéaire ou cyclique, ramifié ou non, aromatique ou non, comportant ou non un ou plusieurs hétéroatomes et possédant de 1 à 40 atomes de carbone,
Z4 désignant un atome d'hydrogène ou un radical alkyle en C₁C₄ ou un radical acyle en C₁C₃ étant entendu qu'au moins un des radicaux Z4 des radicaux Z, Z', Z2 et Z3 est différent de acyle.

De préférence Z4 désigne un atome d'hydrogène ou un radical méthyle.

Encore plus préférentiellement t=0 et Z, Z' et Z3 désignent -CH2CH2-, et l'un au moins de y ou y' est différent de 0. Les composés de formule (XVII) sont alors des polyéthylèneglycols.

Les polymères aminoplaste éther de formule (I) selon l'invention sont en particulier décrits dans le brevet US 5 914 373 dont le contenu fait partie intégrante de l'invention.

Comme polymères à squelette aminoplaste-éther de formule (I), on peut en particulier citer les produits PURE-THIX L [PEG-180/Octoxynol-40/TMMG Copolymer (Nom INCI)], PURE-THIX M [PEG-180/Laureth-50/TMMG Copolymer (Nom INCI)], et PURE-THIX HH [Polyether-1 (Nom INCI)] vendus par la société SUD-CHEMIE ou le PURE-THIX TX1442,de structure tetrakis méthoxy méthyl glycoluril/polyéther modifié hydrophobe.

Les polymères à squelette aminoplaste-éther sont utilisés de préférence en une quantité pouvant varier d'environ 0,05 à 3% en poids du poids total de la composition. Plus préférentiellement, cette quantité varie d'environ 0,3 à 2% en poids.

Les compositions conformes à l'invention peuvent comprendre un polymère fixant.

Les polymères fixants cationiques, anioniques, amphotères et non ioniques utilisables conformément à l'invention sont décrits ci-après.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 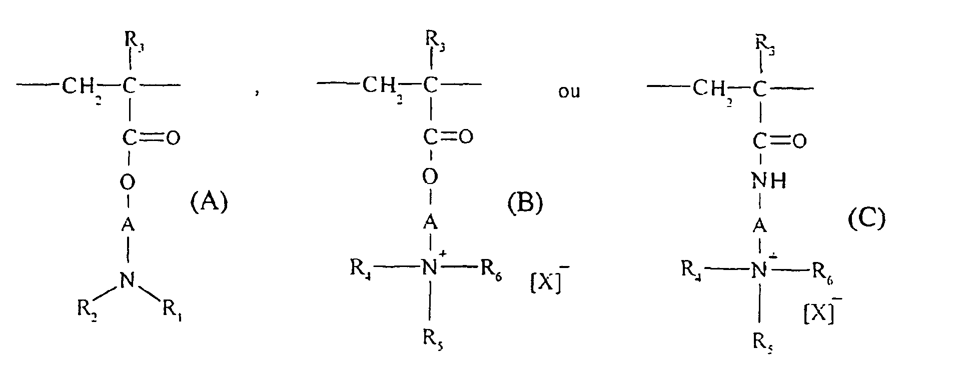 dans lesquelles:
   R₃ désigne un atome d'hydrogène ou un radical CH₃;
   A est un groupe alkyle linéaire ou ramifié de 1 à 6 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle;
   R₁ et R₂ représentent hydrogène ou un groupe alkyle ayant de 1 à 6 atomes de carbone;
   X désigne un anion méthosulfate ou un halogénure tel que chlorure ou bromure.

Les copolymères de la famille (1) contiennent en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone ou le vinylcaprolactame, des esters vinyliques.

Ainsi, parmi ces copolymères de la famille (1), on peut citer :
- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/vinylpyrrolidone tel que le produit vendu sous la dénomination GAFFIX VC 713 par la société ISP,
- et le copolymère vinylpyrrolidone / méthacrylamide de diméthylaminopropyle quaternisé tel que le produit vendu sous la dénomination "GAFQUAT HS 100" par la société ISP.

(2) les polysaccharides quaternisés décrits plus particulièrement dans les brevets américains 3.589.578 et 4.031.307 tel que les gommes de guar contenant des groupements cationiques trialkylammonium.

De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole tels que les produits commercialisés par BASF sous l'appellation Luviquat TFC;
(4) les chitosanes ou leurs sels;

les sels utilisables sont en particulier les acétate, lactate, glutamate, gluconate ou le pyrrolidone carboxylate de chitosane .
Parmi ces composés, on peut citer le chitosane ayant un taux de désacétylation de 90,5% en poids vendu sous la dénomination KYTAN BRUT STANDARD par la société ABER TECHNOLOGIES, le pyrrolidone carboxylate de chitosane vendu sous la dénomination KYTAMER PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants anioniques généralement utilisés sont des polymères comportant des groupements dérivés d'acide carboxylique, sulfonique ou phosphorique et ont un poids moléculaire compris entre environ 500 et 5.000.000.
1) Les groupements carboxyliques sont apportés par des monomères mono ou diacides carboxyliques insaturés tels que ceux répondant à la formule : 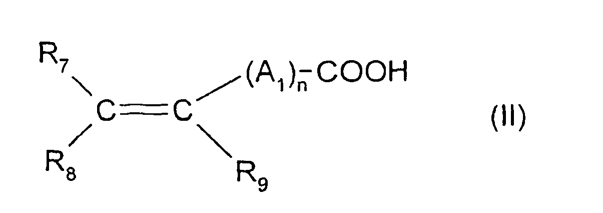 dans laquelle n est un nombre entier de 0 à 10, A₁ désigne un groupement méthylène, éventuellement relié à l'atome de carbone du groupement insaturé
   ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, Rg désigne un atome d'hydrogène, un groupement alkyle inférieur, un groupement -CH₂-COOH, phényle ou benzyle ;

Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/acrylate de tertiobutyle tel que le produit vendu sous la dénomination LUVIMER 100 P par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α-ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. ; De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
      Ces polymères sont par exemple décrits dans les brevets français 2.350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère acétate de vinyle/acide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.

Les polymères fixants anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes;
B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.

Les comonomères basiques préférés sont des méthacrylates d'aminoéthyle, de butyl aminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) les polyamino amides réticulés et alcoylés partiellement ou totalement dérivant de polyaminoamides de formule générale III: 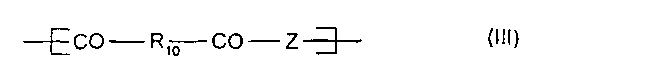 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical IV 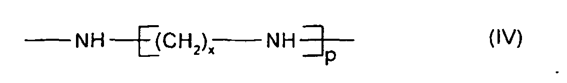 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (IV) ci-dessus, dans lequel x=2 et p=1 et qui dérive de l'éthylènediamine, ou le radical dérivant de la pipérazine : 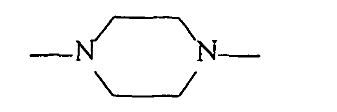
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanes sultones utilisées dans l'alcoylation sont de préférence la propane ou la butane sultone, les sels des agents d'alcoylation sont de préférence les sels de sodium ou de potassium.
(4) les polymères comportant des motifs zwittérioniques de formule V: 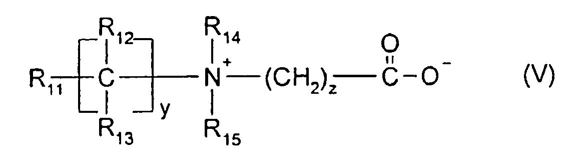 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.

Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle. A titre d'exemple, on peut citer le copolymère de méthacrylate de méthyle /diméthyl carboxyméthylammonio éthylméthacrylate de méthyle tel que le produit vendu sous la dénomination DIAFORMER Z301 par la société SANDOZ.
(5) les polymères dérivés du chitosane comportant des motifs monomères répondant aux formules suivantes : 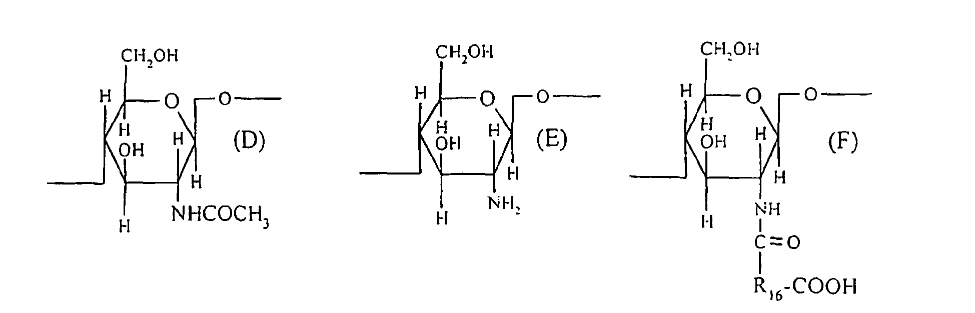 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 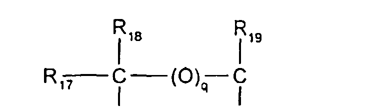 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (VI) par exemple décrits dans le brevet français 1 400 366 : 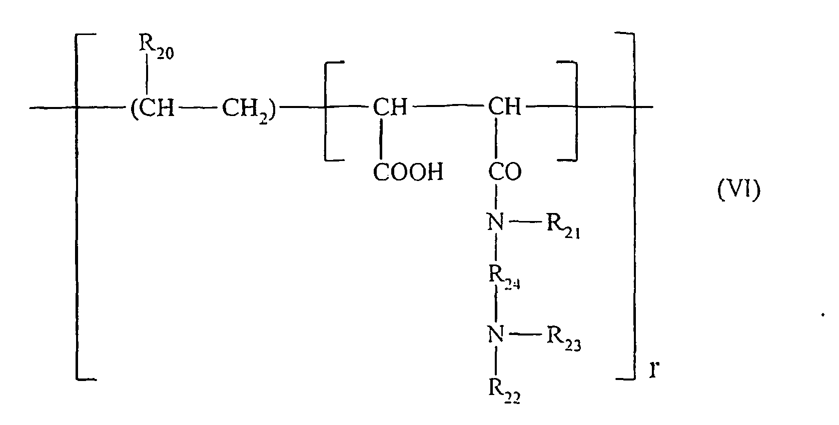 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂- , -CH₂-CH₂-CH₂- , -CH₂-CH(CH₃)- , R₂₂ ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (VII)

      où D désigne un radical 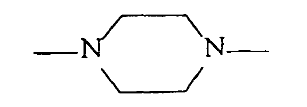 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -D-X-D-X- (VII')

      où D désigne un radical 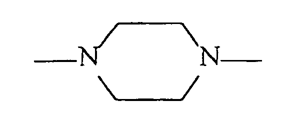 et X désigne le symbole E ou E' et au moins une fois E': E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71
ou LOVOCRYL 47 par la société NATIONAL STARCH et ceux de la famille (4) tels que les copolymère de méthacrylate de méthyle / diméthyl carboxyméthylammonio éthylméthacrylate de méthyle par exemple vendu sous la dénomination DIAFORMER Z301 par la société SANDOZ.

Les polymères fixants non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
- les gommes de guar non ioniques chimiquement modifiées ou non modifiées. Les gommes de guar non ioniques non modifiées sont par exemple les produits vendus sous la dénomination VIDOGUM GH 175 par la société UNIPECTINE et sous la dénomination JAGUAR C par la société MEYHALL.

Les gommes de guar non-ioniques modifiées utilisables selon l'invention sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.

Les radicaux alkyle des polymères non ioniques ont de 1 à 6 atomes de carbone sauf mention contraire.

Les polymères non ioniques qui conviennent tout particulièrement pour la réalisation des compositions conformes à l'invention sont ceux choisis parmi:
* les copolymères de vinyllactame tels que les copolymères de vinylpyrrolidone et d'acétate de vinyle et les copolymères vinylpyrrolidone/acétate de vinyle/ propionate de vinyle
* la polyvinylcaprolactame LUVISKOL PLUS (BASF)
* les homopolymères d'acétate de vinyle tels que APPRETAN EM (HOECHST) ou RHODOPAS A 012 (RHONE POULENC)
* les polyalkyloxazolines tels que PEOX 50 000 et PEOX 500 000 (DOW CHEMICAL)
* les copolymères d'acétate de vinyle et d'ester acrylique tels que le RHODOPAS AD 310 (RHONE POULENC)
* les copolymères d'acétate de vinyle et d'éthylène tels que APPRETAN TV (HOECHST)
* les copolymères d'acétate de vinyle et d'ester maléique tels que APPRETAN MB EXTRA (HOECHST)
* les homopolymères d'acrylates d'alkyle et les homopolymères de métacrylates d'alkyle tels que LUHYDRAN A 848 S (BASF)
* les copolymères d'esters acryliques tels que PRIMAL AC-261 K (ROHM & HAAS), ACRONAL 601 (BASF) ou APPRETAN N 9213 (HOECHST)
* les copolymères d'acrylonitrile et d'un monomère non-ionique tels que CJ 0601 B (ROHM & HAAS)
* les polyuréthanes tels que ACRYSOL RM 1020 ou ACRYSOL RM 2020 (ROHM & HAAS)
* les copolymères d'acrylate d'alkyle et d'uréthane tels que 8538-33 (NATIONAL STARCH)
* les polyamides tels que ESTAPOR LO 11 (RHONE POULENC)

Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 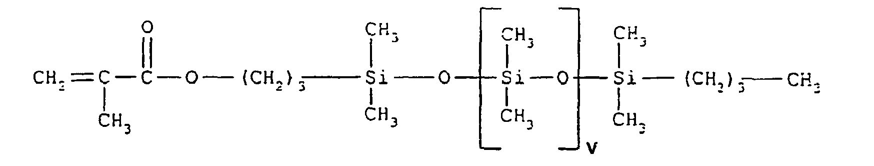 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex Silk, commercialisé par la Société BASF.

On peut aussi utiliser, comme polymères fixants, des polyuréthanes fonctionnalisés ou non, siliconés ou non.

Les polyuréthanes particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0 619 111 de la Société National Starch.

Comme polyuréthanne convenant particulièrement bien pour l'invention, on peut citer les produits commercialisés sous les dénominations Luviset PUR et Luviset Si-PUR par la Société BASF.

La concentration relative en poids en polymère fixant dans la composition est avantageusement comprise entre 0,05 et 20 % en poids, et plus avantageusement encore entre 0,5 et 10 %.

Les compositions conformes à l'invention peuvent comprendre un agent de conditionnement.

On peut choisir avantageusement un agent de conditionnement dans le groupe comprenant les poly-α-oléfines, les huilés fluorées, les huiles végétales, les cires naturelles, les cires fluorées, les gommes fluorées et les esters d'acides gras, les organosiloxanes, les composés amides comportant au moins une chaîne grasse, les céramides ; lesdits agents pouvant être présents sous forme de mélanges.

Parmi les organosiloxanes utilisés conformément à la présente invention, on peut citer à titre non limitatif :

### 1. Les silicones volatiles

Celles-ci possèdent un point d'ébullition compris entre 60°C et 260°C. Parmi ce type de silicones, on cite :
(i) les silicones cycliques de 3 à 7 atomes de silicium et de préférence 4 à 5. Il s'agit, par exemple, de l'octaméthylcyclo-tétrasiloxane vendu sous le nom de "VOLATILE SILICONE 7207®" par la Société UNION CARBIDE ou "SILBIONE 70045 V2®" par la Société RHONE POULENC, le décaméthylcyclopentasiloxane vendu sous le nom de "VOLATILE SILICONE 7158®" par la Société UNION CARBIDE, "SILBIONE 70045 V5®" par la Société RHONE POULENC, ainsi que leurs mélanges.
   On cite également les cyclopolymères du type diméthylsiloxane/méthylalkylsiloxane, tel que la "SILICONE VOLATILE FZ 3109®" vendue par la Société UNION CARBIDE, qui est un cyclocopolymère diméthylsiloxane/méthyloctylsiloxane ;
(ii) les silicones volatiles linéaires ayant 2 à 9 atomes de silicium et possédant une viscosité inférieure ou égale à 5.10⁻⁶ m²/s à 25°C. Il s'agit, par exemple, de l'hexaméthyldisiloxane vendu sous la dénomination "SILBIONE 70041 V0,65®" par la Société RHONE POULENC. Ce type de produit est décrit dans l'article de TODD & BYERS "Volatile silicone fluides for cosmetics", Cosmetics and Toiletries, Vol. 91, Jan 76, pages 27-32.

### II. Les silicones non volatiles

Elles sont constituées principalement par les polyalkyl (C1-C35)-siloxanes, les polyarylsiloxanes, les polyalkyl (C1-C35)arylsiloxanes, les gommes et résines de silicone et les polysiloxanes organomodifiés, ainsi que leurs mélanges.

Parmi les polyalkylsiloxanes, on peut citer principalement les polydiméthylsiloxanes linéaires de viscosité supérieure à 5.10⁻⁶ m²/s, et de préférence inférieure à 2,6 m²/s soit :
- à groupements terminaux triméthylsilyle, comme par exemple, et à titre non limitatif, les huiles "SILBIONE®" de la série 70047 commercialisées par la Société RHONE POULENC, l'huile "47 V 500.000®" de RHONE POULENC ou certaines "VISCASIL®" de la Société GENERAL ELECTRIC,
- à groupements terminaux trihydroxysilyle, tels que les huiles de la série "48 V®" de la Société RHONE POULENC.

Dans cette classe de polyalkylsiloxanes, on peut également mentionner les polyalkylsiloxanes vendus par la Société GOLDSCHMIDT sous les dénominations "ABILWAX 9800®" et "ABILWAX 9801®", qui sont des polyalkyl(C₁-C₂₀)siloxanes.

Parmi les polyalkylarylsiloxanes, on peut citer les polydiméthylphénylsiloxanes, les polydiméthyldiphénylsiloxanes linéaires et/ou ramifiés, de viscosité 10⁻⁵ à 5.10-2 m²/s, tels que par exemple :
- l'huile "RHODORSIL®" 763 de RHONE POULENC,
- les huiles "SILBIONE®" de la série 70641 de RHONE POULENC, telles que les huiles "SILBIONE 70641 V30®" et "SILBIONE 70641 V200®" de RHONE POULENC,
- le produit "DC 556®" Cosmetic Grad Fluid de DOW CORNING,
- les silicones de séries PK de BAYER, telles que la "PK20®",
- les silicones des séries PN, PH de BAYER, comme les "PN 1000®" et "PH 1000®",
- certaines huiles des séries SF de GENERAL ELECTRIC, telles que les "SF 1250®", "SF 1265®", "SF 1154®", "SF 1023®".

Les gommes de silicone, conformes à la présente invention, sont des polydiorganosiloxanes de forte masse moléculaire moyenne en nombre comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.

On cite, par exemple, les composés ayant les structures suivantes:
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/ (méthylvinylsiloxane)].

On peut citer, par exemple, à titre non limitatif, les mélanges suivants :
1) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401®" vendu par la Société DOW CORNING ;
2) les mélanges formés à partir d'une gomme polydiméthylsiloxane avec une silicone cyclique, tel que le produit "SF 1214 SILICONE FLUID®" de GENERAL ELECTRIC, qui est une gomme "SE 30®" de PM 500.000 (Mn) solubilisée dans la "SF 1202 SILICONE FLUID®" (décaméthylcyclopentasiloxane) ;
3) les mélanges de deux PDMS de viscosités différentes, notamment d'une gomme PDMS et d'une huile PDMS, tels que les produits "SF 1236®" et "CF 1241®" de la Société GENERAL ELECTRIC. Le produit "SF 1236®" est le mélange d'une gomme "SE 30®" définie ci-dessus d'une viscosité de 20 m²/s et d'une huile "SF 96®" d'une viscosité de 5.10⁻⁶ m²/s (15 % de gomme "SE 30®" et 85 % d'huile "SF 96®").

Le produit "CF 1241®" est le mélange d'une gomme "SE 30®" (33 %) et d'une PDMS (67 %) de viscosité 10⁻³ m²/s.

Les résines d'organopolysiloxanes utilisables conformément à l'invention, sont des systèmes siloxaniques réticulés renfermant les unités : R₂SiO_{2/2}, RSiO_{3/2} et SiO_{4/2} dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

Parmi ces résines, on peut citer le produit vendu sous la dénomination "DOW CORNING 593®" ou ceux vendus sous les dénominations "SILICONE FLUID SS 4230" et "SILICONE FLUID SS 4267" par la Société GENERAL ELECTRIC et qui sont des diméthyl/triméthylpolysiloxanes.

Les silicones organomodifiées, conformes à la présente invention, sont des silicones telles que définies précédemment, comportant dans leur structure générale, un ou plusieurs groupements organofonctionnels directement fixés sur la chaîne siloxanique ou fixés par l'intermédiaire d'un radical hydrocarboné.

On cite, par exemple, les silicones comportant :
a) des groupements perfluorés tels que des trifluoroalkyles comme, par exemple, celles vendues par la Société GENERAL ELECTRIC sous les dénominations "FF.150 FLUOROSILICONE FLUID®" ou par la Société SHIN ETSU sous les dénominations "X-22-819®", "X-22-82®", "X-22-821®" et "X-22-822®" ;
b) des groupements hydroxyacylamino comme, par exemple, celles décrites dans la demande de brevet EP-A-0 342 834 et en particulier la silicone vendue par la Société DOW CORNING sous la dénomination "Q2-8413®" ;
c) des groupements thiols comme dans les silicones "X 2-8360®" de la Société DOW CORNING ou les "GP 72A®" et "GP 71®" de GENESEE ;
d) des groupements aminés substitués ou non, comme dans la "GP 4 SILICONE FLUID®" de GENESEE, la "GP 7100®" de GENESEE, la "Q2 8220®" de Dow CORNING, I"'AFL 40®" d'UNION CARBIDE ou la silicone dénommée "Amodiméthicone" dans le dictionnaire CTFA ;
e) les groupements carboxylates, comme les produits décrits dans le brevet EP 186 507 de CHISSO CORPORATION ;
f) des groupements hydroxylés, comme les polyorgano-siloxanes à fonction hydroxyalkyle, décrits dans la demande de brevet FR-85 16334, répondant à la formule suivante : 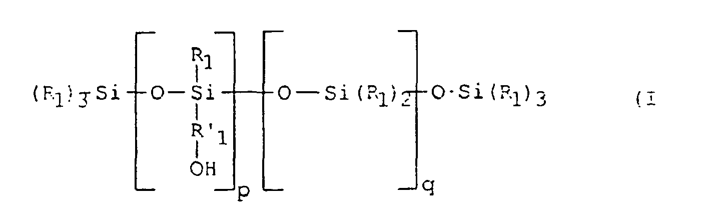 dans laquelle :
   - les radicaux R₁, identiques ou différents, sont choisis parmi les radicaux méthyle et phényle, au moins 60 % en mole des radicaux R₁ étant méthyle ;
   - le radical R'₁ est un chaînon alkylène divalent hydrocarboné en C₂-C₁₈ ;
   - p est compris entre 1 et 30 inclus ;
   - q est compris entre 1 et 150 inclus.

On peut citer tout particulièrement le produit commercialisé par Dow Corning sous l'appellation DC 190 ;
g) des groupements alcoxylés comme dans la Silicone copolymer "F 755®" de SWS SILICONES et les produits "ABILWAX 2428®", "ABILWAX 2434®", "ABILWAX 2440®" de la Société GOLDSCHMIDT ;
h) des groupements acyloxyalkyles, comme par exemple les polyorganopolysiloxanes décrits dans la demande de brevet FR-88 17433, répondant à la formule suivante : 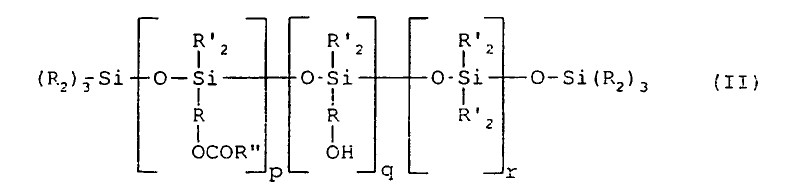 dans laquelle :
   - R₂ désigne méthyle, phényle, OCOR", hydroxyle, un seul des R₂ par atome de silicium peut être OH ;
   - R'₂ désigne méthyle, phényle, 60 % molaire au moins de l'ensemble des radicaux R₂ et R'₂ est méthyle ;
   - R" désigne alcoyle ou alcényle en C₈-C₂₀;
   - R désigne un alkylène hydrocarboné divalent, linéaire ou ramifié en C₂-C₁₈;
   - r est compris entre 1 et 120 inclus ;
   - p est compris entre 1 et 30 inclus ;
   - q vaut 0 ou est inférieur à 0,5 p, p + q étant compris entre 1 et 30 inclus :
      les polyorganosiloxanes de formule (II) peuvent contenir des groupements 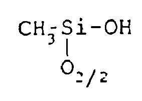 dans des proportions ne dépassant pas 15 % de la somme p + q + r ;
i) des groupements ammonium quaternaires, comme dans les produits "X2 81 08®" et "X2 81 09®", le produit "ABIL K 3270®" de la Société GOLDSCHMIDT;
j) des groupements amphotères ou bétaïniques, tels que dans le produit vendu par la Société GOLDSCHMIDT sous la dénomination "ABIL B 9950®" ;
k) des groupements bisulfites, tels que dans les produits vendus par la Société GOLDSCHMIDT SOUS les dénominations "ABIL S 201®" et "ABIL S 255®" ;
I) des groupements oxyalkylénés.

Les viscosités des silicones peuvent être notamment déterminées par la norme ASTM D445-97 (viscométrie).

Lorsque l'agent de conditionnement de la composition selon l'invention est un hydrocarbure, celui-ci est un hydrocarbure, linéaire ou ramifié en C₈-C₃₀₀. Parmi les hydrocarbures liquides à température ambiante répondant à cette définition, on peut notamment citer l'isododécane, l'isohexadécane et ses isomères (tels que le 2,2,4,4,6,6-heptaméthylnonane), l'isoeicosane, l'isotétracosane, et les isomères desdits composés. On utilise de préférence selon l'invention l'isododécane ou l'un de ses isomères.

Lorsque l'agent de conditionnement est un alcool gras, celui-ci est du type linéaire ou ramifié, saturé ou insaturé, en C₈-C₃₀ et parmi ceux-ci on peut citer le butyl-2 octanol, l'alcool laurique, l'alcool oléique, l'alcool isocétylique. l'alcool isostéarique et l'alcool béhénique.

Lorsque l'agent de conditionnement est un ester gras, celui-ci peut être soit un ester d'un acide gras en C₈-C₃₀ et d'alcool en C₁-C₃₀ soit un ester d'un acide ou diacide en C₁-C₇ et d'un alcool gras en C₈-C₃₀. Parmi ces esters on peut citer le palmitate d'éthyle, d'isopropyle, d'éthyl-2-hexyle et de 2-octyldécyle, le myristate d'isopropyle, de butyle, de cétyle et de 2-octyldécyle, le stéarate de butyle et d'hexyle, le laurate d'hexyle et de 2-hexyldécyle, l'isononanoate d'isononyle et le malate de dioctyle.

Les céramides ou analogues, tels que les glycocéramides utilisables dans les compositions selon l'invention, sont connus en eux-mêmes et sont des molécules naturelles ou synthétiques pouvant répondre à la formule générale suivante : 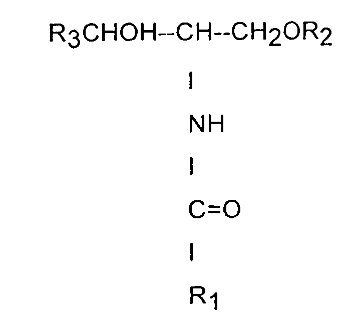 dans laquelle :
- R₁ désigne un radical alkyle, linéaire ou ramifié, saturé ou insaturé, dérivé d'acides gras en C₁₄-C₃₀, ce radical pouvant être substitué par un groupement hydroxyle en position α, ou un groupement hydroxyle en position ω estérifié par un acide gras saturé ou insaturé en C₁₆-C₃₀ ;
- R₂ désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R₃ désigne un radical hydrocarboné en C₁₅-C₂₆, saturé ou insaturé en position α, ce radical pouvant être substitué par un ou plusieurs radicaux alkyle en C₁-C₁₄ ;
étant entendu que dans le cas des céramides ou glycocéramides naturelles, R₃ peut également désigner un radical α-hydroxyalkyle en C₁₅-C₂₆, le groupement hydroxyle étant éventuellement estérifié par un α-hydroxyacide en C₁₆-C₃₀.

Les céramides préférées dans le cadre de la présente invention sont celles décrites par DOWNING dans Arch. Dermatol, Vol. 123, 1381-1384, 1987, ou celles décrites dans le brevet français FR-2 673 179, dont les structures peuvent être les suivantes :

Les céramides plus particulièrement préférées selon l'invention sont les composés pour lesquels R₁ désigne un alkyle saturé ou insaturé dérivé d'acides gras en C₁₆-C₂₂ ; R₂ désigne un atome d'hydrogène ; et R₃ désigne un radical linéaire saturé en C₁₅.

De tels composés sont par exemple :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
ou les mélanges de ces composés.

Encore plus préférentiellement, on utilise les céramides pour lesquels R₁ désigne un radical alkyle saturé ou insaturé dérivé d'acides gras ; R₂ désigne un radical galactosyle ou sulfogalactosyle ; et R₃ désigne un groupement

-CH=CH-(CH₂)₁₂-CH₃.

A titre d'exemple, on peut citer le produit constitué d'un mélange de ces composés, vendu sous la dénomination commerciale GLYCOCER par la société WAITAKI INTERNATIONAL BIOSCIENCES.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

Parmi ces alcools, on peut citer l'éthanol, l'isopropanol. L'éthanol est particulièrement préféré.

De préférence, la concentration en solvant(s) organique est inférieure à 30 %, et plus préférentiellement encore inférieure à 20 %.

La composition de l'invention peut également contenir au moins un additif choisi parmi les tensioactifs non ioniques, anioniques, cationiques, amphotères ou zwitterioniques, les parfums, les filtres, les conservateurs, les protéines, les vitamines, les polymères non ioniques, anioniques, cationiques, amphotères ou zwitterioniques autres que ceux de l'invention, les huiles minérales, végétales ou synthétiques, les agents épaississants et tout autre additif classiquement utilisé dans les compositions cosmétiques, les agents antipellicullaires, les agents anti-chute, les électrolytes, les colorants, les pigments, les agents hydratants, tels que la glycérine et les autres polyols, les réducteurs.

Ces additifs sont éventuellement présents dans la composition selon l'invention dans des proportions pouvant aller, avantageusement, de 0,001 à 20% en poids par rapport au poids total de la composition. La quantité précise de chaque additif est fonction de sa nature et est déterminée facilement par l'homme de l'art, et dépendra de l'application capillaire choisie.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions de l'invention peuvent être utilisées pour la fabrication de nombreux produits capillaires, comme, par exemple, des produits pour la fixation et/ou le maintien des cheveux, des produits de conditionnement, comme des formulations de brillance ou encore des produits de soin des cheveux.

Ces formulations capillaires selon l'invention seront conditionnées en aérosol ou en flacon pompe, sans propulseur.

L'invention pourra être mieux comprise à l'aide des exemples non limitatifs qui suivent et qui constituent des modes de réalisation avantageux des compositions conformes à l'invention.

Toutes les quantités sont exprimées en pourcentages relatifs en poids par rapport au poids total de la composition et m.a. signifie matière active.

### EXEMPLES :

On réalise les compositions suivantes conformes à l'invention. La formule B est une composition aqueuse, et la formule D, une composition hydroalcoolique, à fortes teneurs en éthanol. Les compositions E, F et G contiennent, outre un polymère épaississant à squelette aminoplaste-éther, soit un agent de conditionnement, soit un polymère fixant.

### Mousse en solvant aqueux sans propulseur

La composition suivante est conditionnée dans un équipement Pompe mousse réf F2 commercialisé par AIRSPRAY.

### Formule B

| | | |
|---|---|---|
| Pure thix TX1442 | | 0.3% |
| Eau | qsp | 100g |

Les mousses obtenues à partir de la formule B est agréable, de densité 0,19 g/cm³, la densité étant mesurée selon la méthode exposée dans le brevet FR 2 704 771 dont la Demanderesse est Titulaire.

### Mousse en solvant hydroalcoolique sans propulseur

On utilise à nouveau l'équipement Pompe mousse réf F2 de chez AIRSPRAY.

### Formule D

| | | |
|---|---|---|
| Pure thix TX1442 | | 1% |
| Ethanol | | 30% |
| Eau | qsp | 100g |

Les mousses obtenues à partir de la formule D est agréable malgré sa forte teneur en alcool, de densité 0,12 g/cm³, la densité étant mesurée selon la méthode exposée dans le brevet FR 2 704 771 dont la Demanderesse est Titulaire.

On réalise d'autres compositions comprenant cette fois, outre un polymère conforme à l'invention, un polymère fixant ou un agent de conditionnement.

### Mousse avec polymère fixant anionique:

### Formule E

| | | |
|---|---|---|
| Pure thix TX1442 | | 0.5% |
| ACRYLIDONE LM commercialisé par ISP | | 0.5% |
| Amino-Méthyl-Propanol | qsp neutralisation | à 100% |
| Eau | qsp | 100% |

### Mousses avec agents de conditionnement

### Formule F:

| | | |
|---|---|---|
| Pure thix TX1442 | | 1 % |
| LUVIQUAT FC 370 commercialisé par BASF | | 2.5% |
| Eau | qsp | 100% |

### Formule G :

| | | |
|---|---|---|
| Pure thix TX1442 | | 1% |
| DC 939 EMULSION commercialisé par Dow Corning | | 1.43% |
| Eau | qsp | 100% |

Les formules E, F et G donnent des mousses de bonnes qualités, malgré la faible quantité d'agent moussant, de densité proche de 0,1 g/cm³, la densité étant mesurée selon la méthode exposée dans le brevet FR 2 704 771 dont la Demanderesse est Titulaire.

## Revendications

1. Composition cosmétique capillaire sous forme de mousse conditionnée en aérosol ou flacon pompe sans propulseur comprenant, dans un milieu cosmétiquement acceptable, au moins un polymère épaississant à squelette aminoplaste-éther.

2. Composition selon la revendication 1, **caractérisée par le fait que** le ou les polymères aminoplaste-éther sont choisis parmi ceux contenant au moins un motif de structure (I) suivante : 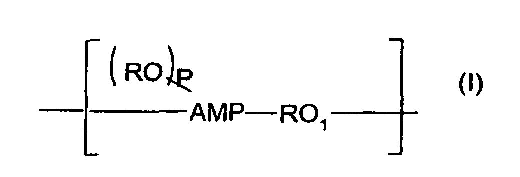 dans laquelle :
- AMP est un résidu aminoplaste avec unités alkylènes,
- R désigne un atome d'hydrogène, un radical alkyl C₁ C₄ ou un radical acyle C₁C₄,
- RO₁ est un résidu alkylèneoxy divalent,
- p désigne un nombre entier positif,
le ou les groupements OR étant liés aux unités alkylènes du résidu AMP.

3. Composition selon les revendications 1 ou 2, **caractérisée par le fait que** le ou les polymères à squelette aminoplaste-éther sont choisis parmi ceux contenant au moins un motif de structure (II) suivante : 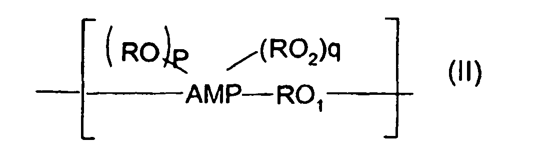 dans laquelle :
- AMP, R, RO₁ et p ont la même signification que dans la revendication 2,
- RO₂ est un groupe hydrophobe différent de RO lié à AMP via un hétéroatome et comprenant au moins deux atomes de carbone, et,
- q est un nombre entier positif.

4. Composition selon la revendication 3, **caractérisée par le fait que** le ou les polymères aminoplaste-éther sont choisis parmi les polymères de structure (III) ou (IIIbis) suivantes : 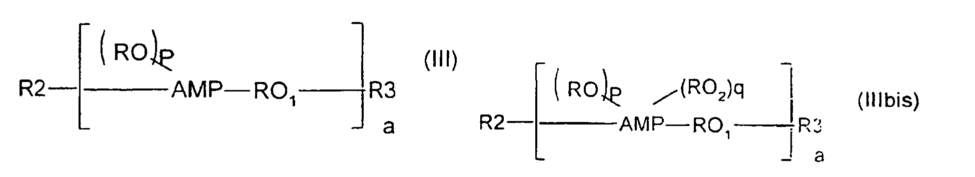 dans lesquelles :
AMP, R, RO₁, RO₂, p et q ont la même signification que dans les revendications 2 et 3,
R2 ou R3, identiques ou différents représentent un groupe terminal pouvant désigner un atome d'hydrogène, un groupement RO₁H, un groupement RO₂H, un groupement AMP(OR)p ou tout groupement monofonctionnel tel que alkyle, cycloalkyle, aryle, aralkyle, alkylaryle, alkyloxyalkyle, aryloxyalkyle, cycloalkoxyalkyle,
a étant un nombre plus grand que 1 et de préférence plus grand que 2.

5. Composition selon l'une quelconque des revendications 2 à 4, **caractérisée**
**par le fait que** les résidus aminoplastes porteurs de leurs groupements OR sont choisis parmi ceux de structures (IV) à (XV) suivantes : 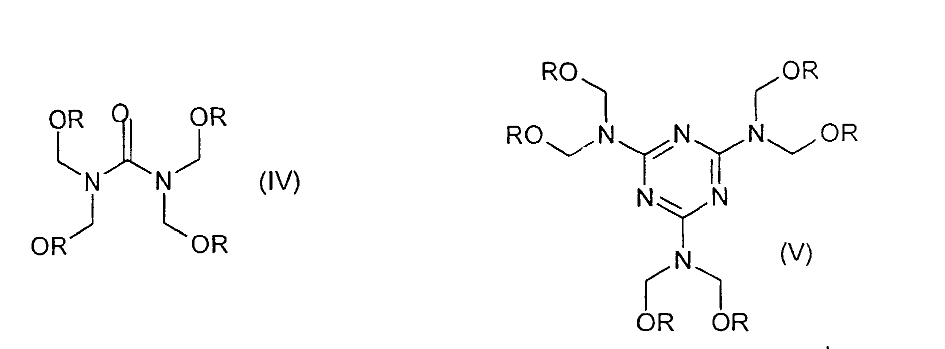 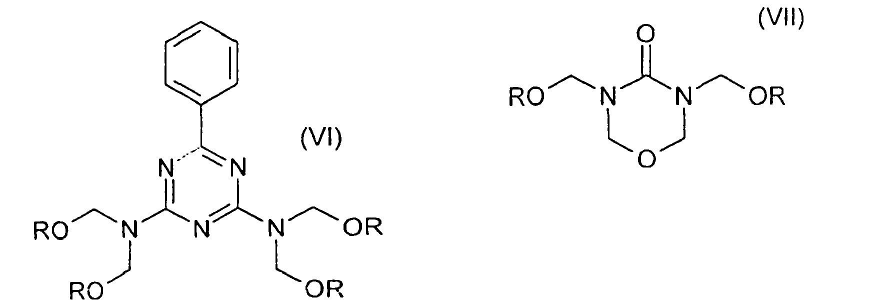 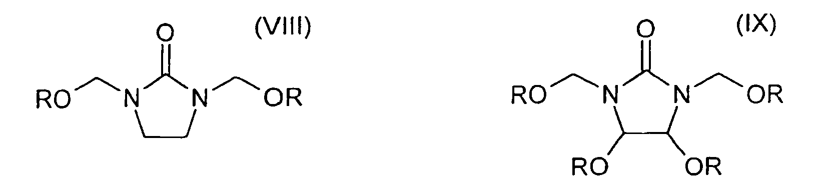 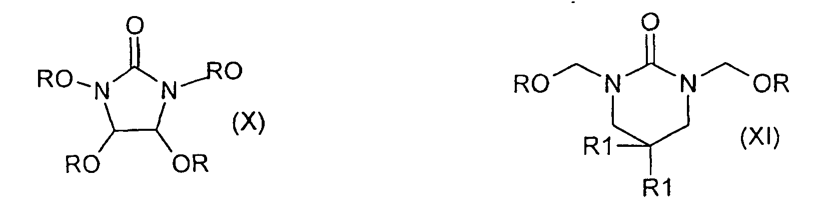 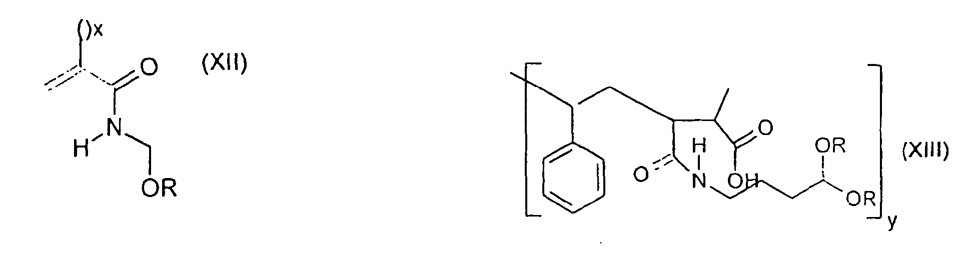 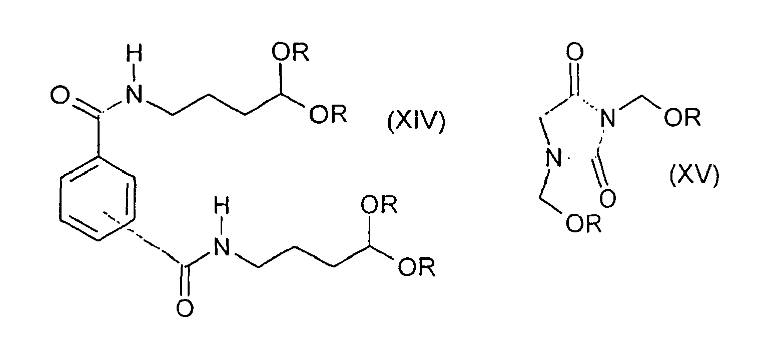
R ayant la même signification que dans la revendication 2,
R1 désigne alkyle C₁C₄,
y est un nombre au moins égal à 2,
x désigne 0 ou 1.

6. Composition selon l'une quelconque des revendications 2 à 4, **caractérisée**
**par le fait que** les résidus aminoplastes porteurs de leurs groupements OR sont choisis parmi ceux de structure (XVI) suivante : 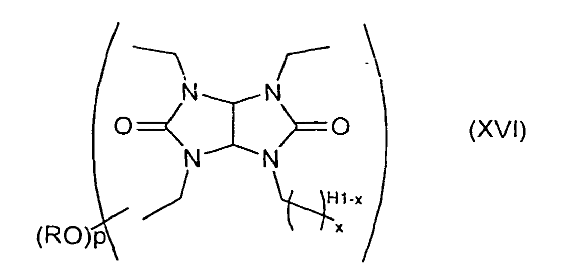 dans laquelle R, p , et x ont les mêmes significations que dans les revendications 2 et 5.

7. Composition selon l'une queconque des revendications 2 à 6, **caractérisée par le fait que** les résidus alkylèneoxy sont ceux correspondants aux diols de formule générale (XVII) suivante :
HO-(ZO)y-(Z1 (Z2O)w)t-(Z'O)y'-Z3OH (XVII),
y et y' étant des nombres allant de 0 à 1000,
t et w étant des nombres allant de 0 à 10,
Z, Z', Z2 et Z3 sont des radicaux alkylène en C₂C₄ et de préférence des radicaux -CH2-CH(Z4)- et -CH2-CH(Z4)-CH2-,
Z1 étant un radical linéaire ou cyclique, ramifié ou non, aromatique ou non, comportant ou non un ou plusieurs hétéroatomes et possédant de 1 à 40 atomes de carbone,
Z4 désignant un atome d'hydrogène ou un radical alkyle en C₁-C₄ ou un radical acyle en C₁C₃ étant entendu qu'au moins un des radicaux Z4 des radicaux Z, Z', Z2 et Z3 est différent de acyle.

8. Composition selon la revendication 7, **caractérisée par le fait que** le radical Z4 désigne un atome d' hydrogène ou un radical méthyle.

9. Composition selon les revendications 7 et 8, **caractérisée par le fait que** t=0 et que Z, Z' et Z3 désignent -CH2CH2- , l'un au moins de y ou y' étant différent de 0.

10. Composition selon l'une quelconque des revendications précédentes,
**caractérisée par le fait que** les polymères à squelette aminoplaste-éther sont choisi parmi les produits suivants :
PEG-180/Octoxynol-40/TMMG Copolymer,
PEG-180/Laureth-50/TMMG Copolymer,
Polyether-1
Tetrakis méthoxy méthyl glycoluril/polyéther modifié hydrophobe.

11. Composition selon l'une quelconque des revendications 1 à 10, **caractérisée par le fait que** les polymères à squelette aminoplaste-éther sont utilisés en une quantité pouvant varier de 0,05 à 3% en poids du poids total de la composition.

12. Composition selon l'une quelconque des revendications 1 à 11, **caractérisée par le fait que** les polymères à squelette aminoplaste-éther sont utilisés en une quantité pouvant varier de 0,3 à 2% en poids du poids total de la composition.

13. Composition selon l'une quelconque des revendications précédentes, **caractérisée par le fait qu'**elle comprend, en outre, au moins un polymère fixant.

14. Composition selon la revendication 13, **caractérisée par le fait que** la concentration relative en poids en polymère(s) fixant(s) est comprise entre 0,05 et 20 % en poids, et de préférence entre 0,5 et 10%.

15. Composition selon les revendications 13 et 14, **caractérisée par le fait que** le polymère fixant est un polymère anionique choisi parmi :
- les polymères comportant des motifs carboxyliques dérivant de monomères mono ou diacides carboxyliques insaturés de formule : 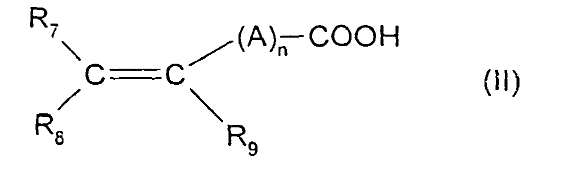 dans laquelle n est un nombre entier de 0 à 10, A désigne un groupement méthylène, éventuellement relié à l'atome de carbone du groupement insaturé
ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
- les polymères comprenant des motifs dérivant d'acide sulfonique tels que des motifs vinylsulfonique, styrènesulfonique, acrylamido alkylsulfonique.

16. Composition selon les revendications 13 et 14, **caractérisée en ce que** le polymère fixant est un polymère amphotère choisi parmi les polymères comportant des motifs dérivant:
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

17. Composition selon les revendications 13 et 14, **caractérisée par le fait que** le polymère fixant est un polymère non ionique choisi parmi :
- les polyalkyloxazolines;
- les homopolymères d'acétate de vinyle;
- les copolymères d'acétate de vinyle et d'ester acrylique;
- les copolymères d'acétate de vinyle et d'éthylène;
- les copolymères d'acétate de vinyle et d'ester maléïque;
- les copolymères de polyéthylène et d'anhydride maléïque;
- les homopolymères d'acrylates d'alkyle et les homopolymères de méthacrylates d'alkyle;
- les copolymères d'esters acryliques tels que par exemple les copolymères d'acrylates d'alkyle et de méthacrylates d'alkyle ;
- les copolymères d'acrylonitrile et d'un monomère non ionique choisi par exemple parmi le butadiène et les (méth)acrylates d'alkyle;
- les copolymères d'acrylate d'alkyle et d'uréthanne ; et
- les polyamides ; et
- les gommes de guar non ioniques chimiquement modifiées ou non modifiées.

18. Composition selon les revendications 13 et 14, **caractérisée par le fait que** le polymère fixant est un polymère cationique choisi parmi :
- le copolymère d'acrylamide et de diméthylaminoéthyl méthacrylate quaternisé au sulfate de diméthyle,
- les copolymères d'acrylamide et de chlorure de méthacryloyloxyéthyltriméthylammonium,
- le copolymère d'acrylamide et de méthosulfate de méthacryloyloxyéthyltriméthylammonium,
- les copolymères vinylpyrrolidone / acrylate ou méthacrylate de dialkylaminoalkyle quaternisés ou non,
- les terpolymère méthacrylate de diméthyl amino éthyle/ vinylcaprolactame/vinylpyrrolidone,
- le copolymère vinylpyrrolidone / méthacrylamide de diméthylaminopropyle quaternisé,
- les polysaccharides quaternisés, tels que les gommes de guar contenant des groupements cationiques trialkylammonium,
- les copolymères quaternaires de vinylpyrrolidone et de vinylimidazole,
- les chitosanes ou leurs sels,
- les dérivés de cellulose cationiques.

19. Composition selon les revendications 13 et 14, **caractérisée par le fait que** le polymère fixant est un polyuréthanne fonctionnalisé ou non, siliconé ou non.

20. Composition selon les revendications 13 et 14, **caractérisée par le fait que** le polymère fixant est un polymère de type siliconé greffé comprenant une portion polysiloxane et une portion constituée d'une chaîne organique non-siliconée, l'une des deux portions constituant la chaîne principale du polymère l'autre étant greffée sur la dite chaîne principale.

21. Composition selon l'une quelconque des revendications précédentes,
**caractérisée par le fait qu'**elle comprend, en outre, un agent de conditionnement dans le groupe comprenant les poly-α-oléfines, les huiles fluorées, les huiles végétales, les cires naturelles, les cires fluorées, les gommes fluorées et les esters d'acides gras, les organosiloxanes, les composés amides comportant au moins une chaîne grasse, les céramides ; lesdits agents pouvant être présents sous forme de mélanges.

22. Composition selon l'une quelconque des revendications précédentes,
**caractérisée par le fait qu'**elle au moins un additif choisi parmi les tensioactifs non ioniques, anioniques, cationiques, amphotères ou zwitterioniques, les parfums, les filtres, les conservateurs, les protéines, les vitamines, les polymères non ioniques, anioniques, cationiques, amphotères ou zwitterioniques autres que ceux de l'invention, les huiles minérales, végétales
ou synthétiques, les agents épaississants et tout autre additif classiquement utilisé dans les compositions cosmétiques, les agents antipellicullaires, les agents anti-chute, les électrolytes, les colorants, les pigments, les agents hydratants, tels que la glycérine et les autres polyols, les réducteurs.

23. Procédé cosmétique capillaire, **caractérisé par le fait qu'**il consiste à appliquer sur les cheveux une composition conforme à l'une quelconque des revendications précédentes.

## Claims

1. Cosmetic hair composition in mousse form, packaged in aerosol form or in a pump-bottle without a propellant, comprising, in a cosmetically acceptable medium, at least one thickening polymer with an aminoplast-ether skeleton.

2. Composition according to Claim 1, **characterized in that** the aminoplast-ether polymer(s) is (are) chosen from those containing at least one unit of structure (I) below: 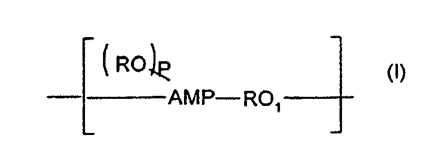 in which:
- AMP is an aminoplast residue with alkylene units,
- R denotes a hydrogen atom, a C₁-C₄ alkyl radical or a C₁-C₄ acyl radical,
- RO₁ is a divalent alkyleneoxy residue,
- p denotes a positive integer,
the group(s) OR being linked to the alkylene units of the AMP residue.

3. Composition according to Claim 1 or 2, **characterized in that** the polymer(s) with an aminoplast-ether skeleton is (are) chosen from those containing at least one unit of structure (II) below: 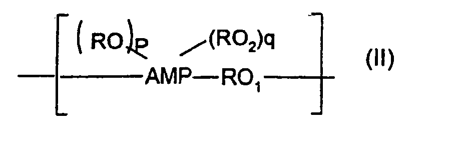 in which:
- AMP, R, RO₁ and p have the same meaning as in Claim 2,
- RO₂ is a hydrophobic group other than RO linked to AMP via a hetero atom and comprising at least two carbon atoms, and
- q is a positive integer.

4. Composition according to Claim 3, **characterized in that** the aminoplast-ether polymer(s) is (are) chosen from the polymers of structure (III) or (IIIa) below: 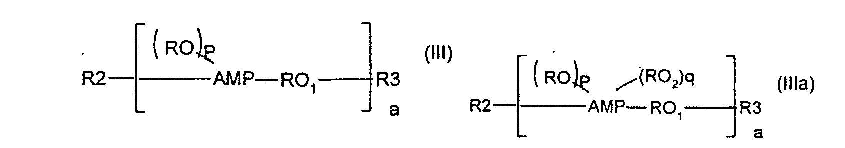 in which:
AMP, R, RO₁, RO₂, p and q have the same meaning as in Claims 2 and 3,
R2 or R3, which may be identical or different, represent an end group that can denote a hydrogen atom, a group RO₁H, a group RO₂H, a group AMP (OR) p or any monofunctional group such as alkyl, cycloalkyl, aryl, aralkyl, alkylaryl, alkyloxyalkyl, aryloxyalkyl or cycloalkoxyalkyl,
a being a number greater than 1 and preferably greater than 2.

5. Composition according to any one of Claims 2 to 4, **characterized in that** the aminoplast residues bearing the groups OR thereof are chosen from those of structures (IV) to (XV) below: 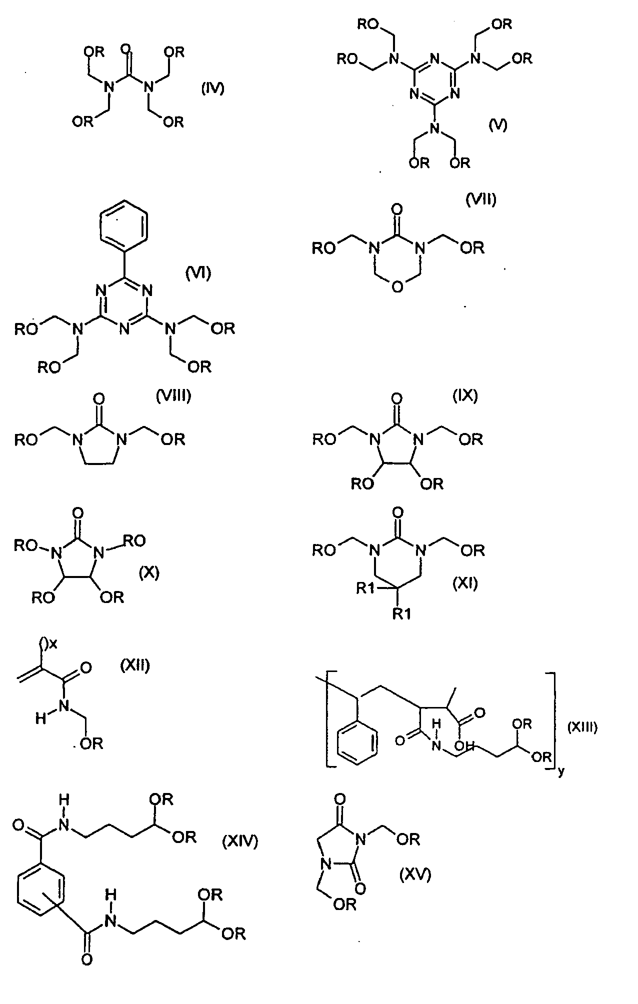
R having the same meaning as in Claim 2,
R1 denotes C₁-C₄ alkyl,
y is a number at least equal to 2,
x denotes 0 or 1.

6. Composition according to any one of Claims 2 to 4, **characterized in that** the aminoplast residues bearing the groups OR thereof are chosen from those of structure (XVI) below: 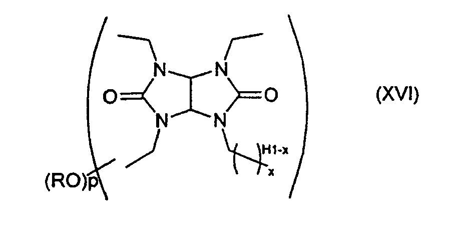 in which R, p and x have the same meanings as in Claims 2 and 5.

7. Composition according to any one of Claims 2 to 6, **characterized in that** the alkyleneoxy residues are those corresponding to the diols of general formula (XVII) below:
HO- (ZO)y- (Z1 (Z2O) w) t- (Z'O)y'-Z3OH (XVII),
y and y' being numbers ranging from 0 to 1000,
t and w being numbers ranging from 0 to 10,
Z, Z', Z2 and Z3 are C₂-C₄ alkylene radicals and preferably radicals -CH2-CH(Z4)- and -CH2-CH(Z4)-CH2-,
Z1 being a linear or cyclic, branched or unbranched, aromatic or nonaromatic radical optionally comprising one or more hetero atoms and containing from 1 to 40 carbon atoms,
Z4 denoting a hydrogen atom or a C₁-C₄ alkyl radical or a C₁-C₃ acyl radical, it being understood that at least one of the radicals Z4 of the radicals Z, Z', Z2 and Z3 is other than acyl.

8. Composition according to Claim 7, **characterized in that** the radical Z4 denotes a hydrogen atom or a methyl radical.

9. Composition according to Claims 7 and 8, **characterized in that** t = 0 and **in that** Z, Z' and Z3 denote -CH2CH2-, at least one from among y and y' being other than 0.

10. Composition according to any one of the preceding claims, **characterized in that** the polymers with an aminoplast-ether skeleton are chosen from the following products:
PEG-180/Octoxynol-40/TMMG Copolymer,
PEG-180/Laureth-50/TMMG Copolymer,
Polyether-1
Tetrakis methoxy methyl glycoluril/hydrophobic modified polyether.

11. Composition according to any one of Claims 1 to 10, **characterized in that** the polymers with an aminoplast-ether skeleton are used in an amount that can range from 0.05% to 3% by weight relative to the total weight of the composition.

12. Composition according to any one of Claims 1 to 11, **characterized in that** the polymers with an aminoplast-ether skeleton are used in an amount that can range from 0.3% to 2% by weight relative to the total weight of the composition.

13. Composition according to any one of the preceding claims, **characterized in that** it also comprises at least one fixing polymer.

14. Composition according to Claim 12, **characterized in that** the relative weight concentration of fixing polymer(s) is between 0.05% and 20% by weight and preferably between 0.5% and 10%.

15. Composition according to Claims 13 and 14, **characterized in that** the fixing polymer is an anionic polymer chosen from:
- polymers comprising carboxylic units derived from unsaturated monocarboxylic or dicarboxylic acid monomers of formula: 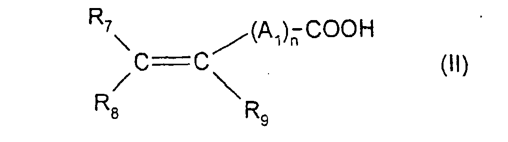
in which n is an integer from 0 to 10, A denotes a methylene group, optionally connected to the carbon atom of the unsaturated group, or to the neighboring methylene group when n is greater than 1, via a hetero atom such as oxygen or sulfur, R₇ denotes a hydrogen atom or a phenyl or benzyl group, R₈ denotes a hydrogen atom or a lower alkyl or carboxyl group, R₉ denotes a hydrogen atom, a lower alkyl group or a -CH₂-COOH, phenyl or benzyl group;
- polymers comprising units derived from sulfonic acid, such as vinylsulfonic, styrenesulfonic or acrylamidoalkylsulfonic units.

16. Composition according to Claims 13 and 14, **characterized in that** the fixing polymer is an amphoteric polymer chosen from polymers comprising units derived from:
a) at least one monomer chosen from acrylamides and methacrylamides substituted on the nitrogen with an alkyl radical,
b) at least one acidic comonomer containing one or more reactive carboxylic groups, and
c) at least one basic comonomer such as esters containing primary, secondary, tertiary and quaternary amine substituents of acrylic and methacrylic acids and the product of quaternization of dimethylaminoethyl methacrylate with dimethyl or diethyl sulfate.

17. Composition according to Claims 13 and 14, **characterized in that** the fixing polymer is a nonionic polymer chosen from:
- polyalkyloxazolines;
- vinyl acetate homopolymers;
- copolymers of vinyl acetate and of acrylic ester;
- copolymers of vinyl acetate and of ethylene;
- copolymers of vinyl acetate and of maleic ester;
- copolymers of polyethylene and of maleic anhydride;
- alkyl acrylate homopolymers and alkyl methacrylate homopolymers;
- acrylic ester copolymers such as, for example, copolymers of alkyl acrylates and of alkyl methacrylates;
- copolymers of acrylonitrile and of a nonionic monomer chosen, for example, from butadiene and alkyl (meth)acrylates;
- copolymers of alkyl acrylate and of urethane;
- polyamides; and
- unmodified or chemically modified nonionic guar gums.

18. Composition according to Claims 13 and 14, **characterized in that** the fixing polymer is a cationic polymer chosen from:
- the copolymer of acrylamide and of dimethylaminoethyl methacrylate quaternized with dimethyl sulfate,
- copolymers of acrylamide and of methacryloyloxyethyltrimethylammonium chloride,
- the copolymer of acrylamide and of methacryloyloxyethyltrimethylammonium methosulfate,
- quaternized or nonquaternized vinylpyrrolidone/dialkylaminoalkyl acrylate or methacrylate copolymers,
- dimethylaminoethyl methacrylate/vinylcaprolactam/vinylpyrrolidone terpolymers,
- the quaternized vinylpyrrolidone/dimethylaminopropylmethacrylamide copolymer,
- quaternized polysaccharides, such as guar gums containing trialkylammonium cationic groups;
- quaternary copolymers of vinylpyrrolidone and of vinylimidazole,
- chitosans or salts thereof,
- cationic cellulose derivatives.

19. Composition according to Claims 13 and 14, **characterized in that** the fixing polymer is a functionalized or unfunctionalized, silicone or nonsilicone polyurethane.

20. Composition according to Claims 13 and 14, **characterized in that** the fixing polymer is a polymer of grafted silicone type comprising a polysiloxane portion and a portion consisting of a nonsilicone organic chain, one of the two portions constituting the main chain of the polymer, the other being grafted onto said main chain.

21. Composition according to any one of the preceding claims, **characterized in that** it also comprises a conditioner from the group comprising poly-α-olefins, fluoro oils, plant oils, natural waxes, fluoro waxes, fluoro gums and fatty acid esters, organosiloxanes, amide compounds comprising at least one fatty chain, and ceramides; said agents possibly being present in the form of mixtures.

22. Composition according to any one of the preceding claims, **characterized in that** it contains at least one additive chosen from nonionic, anionic, cationic, amphoteric or zwitterionic surfactants, fragrances, screening agents, preserving agents, proteins, vitamins, nonionic, anionic, cationic, amphoteric or zwitterionic polymers other than those of the invention, mineral, plant or synthetic oils, thickeners and any other additive conventionally used in cosmetic compositions, antidandruff agents, agents for preventing hair loss, electrolytes, colorants, pigments, moisturizers such as glycerol and other polyols, and reducing agents.

23. Cosmetic hair process, **characterized in that** it consists in applying to the hair a composition in accordance with any one of the preceding claims.

## Patentansprüche

1. Haarkosmetische Zusammensetzung in Form eines Schaums, die ohne Treibmittel in einem Aerosolbehälter oder einem Pumpflakon konfektioniert ist und die in einem kosmetisch akzeptablen Medium mindestens ein verdickendes Polymer mit Aminoplastether-Grundgerüst enthält.

2. Zusammensetzung nach Anspruch 1, **dadurch gekennzeichnet, dass** das oder die Aminoplastether-Polymer(e) unter den Polymeren ausgewählt sind, die mindestens eine Einheit der folgenden Struktur (I) enthalten: 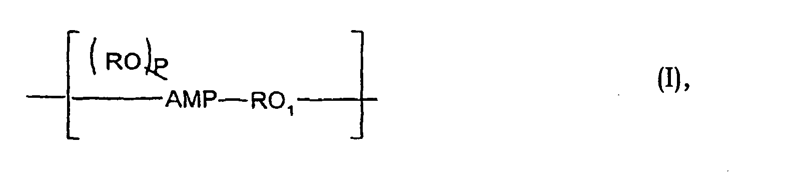 worin bedeuten:
- AMP einen Aminoplastrest mit Alkyleneinheiten,
- R ein Wasserstoffatom, eine C₁₋₄-Alkylgruppe oder eine C₁₋₄₋Acylgruppe,
- RO₁ einen zweiwertigen Alkylenoxyrest,
- p eine positive ganze Zahl,
wobei die Gruppe(n) OR an die Alkyleneinheiten des AMP-Rests gebunden sind

3. Zusammensetzung nach Anspruch 1 oder 2, **dadurch gekennzeichnet, dass** das oder die Polymere(e) mit Aminoplastether-Grundgerüst unter den Polymeren ausgewählt sind, die mindestens eine Einheit der folgenden Struktur (II) enthalten: 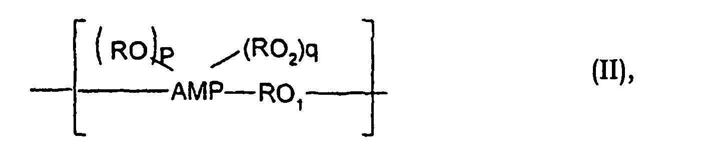 worin bedeuten:
- AMP, R, RO₁ und p die in Anspruch 2 angegebenen Bedeutungen,
- RO₂ eine hydrophobe Gruppe, die von RO verschieden ist und über ein Heteroatom an AMP gebunden ist und mindestens zwei Kohlenstoffatome aufweist, und
- q eine positive ganze Zahl.

4. Zusammensetzung nach Anspruch 3, **dadurch gekennzeichnet, dass** das oder die Aminoplastether-Polymer(e) unter den Polymeren der folgenden Strukturen (III) oder (IIIbis) ausgewählt sind: 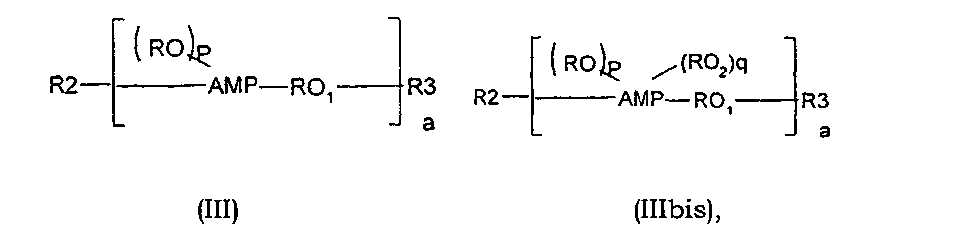 worin bedeuten:
AMP, R, RO₁, RO₂, p und q die in den Ansprüchen 2 und 3 angegebenen Bedeutungen,
R2 oder R3, die gleich oder verschieden sind, eine endständige Gruppe, die ein Wasserstoffatom, eine Gruppe RO₁H, eine Gruppe RO₂H, eine Gruppe AMP(OR)p oder beliebige monofunktionelle Gruppen bedeuten kann, wie Alkyl, Cycloalkyl, Aryl, Aralkyl, Alkylaryl, Alkyloxyalkyl, Aryloxyalkyl, Cycloalkoxyalkyl,
wobei a eine Zahl > 1 und vorzugsweise >2 bedeutet.

5. Zusammensetzung nach einem der Ansprüche 2 bis 4, **dadurch gekennzeichnet, dass** die Aminoplastreste, die Gruppen OR aufweisen, unter den Strukturen der folgenden Formeln (IV) bis (XV) ausgewählt sind: 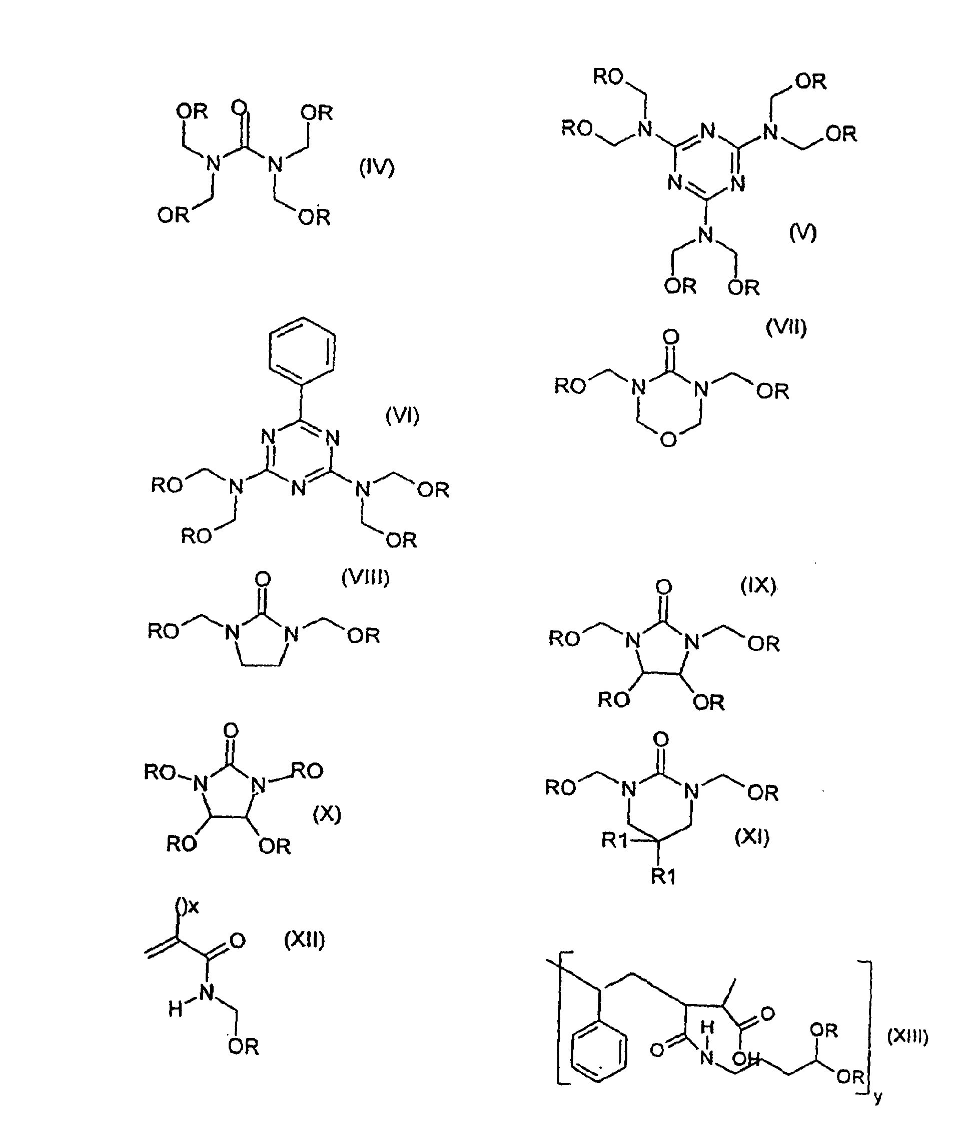 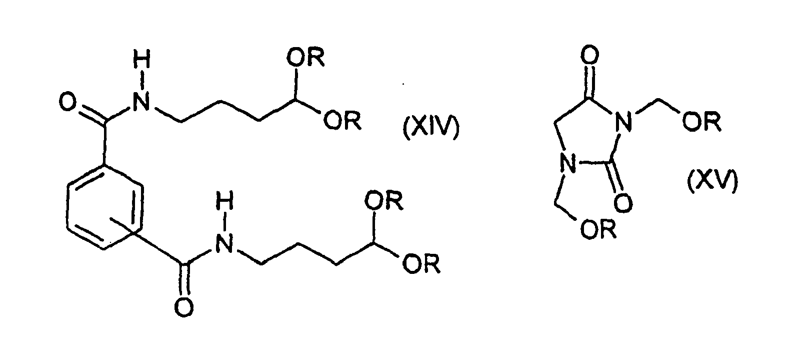
wobei R die in Anspruch 2 angegebene Bedeutung aufweist,
R1 C₁₋₄-Alkyl bedeutet,
y eine Zahl von mindestens 2 ist,
x 0 oder 1 bedeutet.

6. Zusammensetzung nach einem der Ansprüche 2 bis 4, **dadurch gekennzeichnet, dass** die Aminoplastreste, die OR-Gruppen tragen, unter den Resten der folgenden Struktur (XVI) ausgewählt sind: 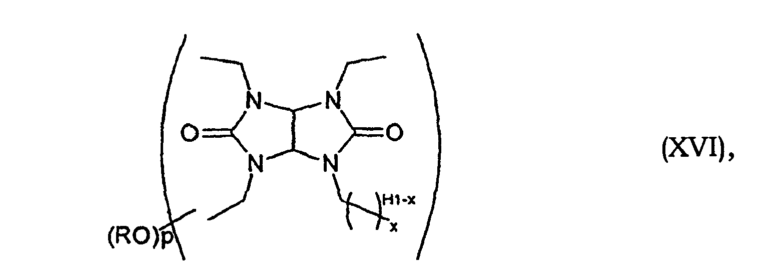 worin R, p und x die in den Ansprüchen 2 und 5 angegebenen Bedeutungen aufweisen.

7. Zusammensetzung nach einem der Ansprüche 2 bis 6, **dadurch gekennzeichnet, dass** die Alkylenoxyreste Gruppen sind, die Diolen der allgemeinen Formel (XVII) entsprechen:
HO-(ZO)y-(Z1 (Z2O)w)t-(Z'O)y'-Z3OH (XVII),
wobei y und y' Zahlen von 0 bis 1 000 bedeuten,
t und w Zahlen von 0 bis 10 bedeuten,
Z, Z', 22 und Z3 C₂₋₄-Alkylengruppen und vorzugsweise die Gruppen -CH2-CH(Z4)- und -CH2-CH(Z4)-CH2- bedeuten,
wobei Z1 eine geradkettige oder cyclische, verzweigte oder nicht verzweigte, aromatische oder nicht aromatische Gruppe ist, die gegebenenfalls ein oder mehrere Heteroatome enthält und 1 bis 40 Kohlenstoffatome aufweist,
Z4 ein Wasserstoffatom, C₁₋₄-Alkyl oder C₁₋₃-Acyl bedeutet, mit der Maßgabe, dass mindestens eine Gruppe Z4 der Gruppen Z, Z', Z2 und Z3 von Acyl verschieden ist.

8. Zusammensetzung nach Anspruch 7, **dadurch gekennzeichnet, dass** die Gruppe Z4 ein Wasserstoffatom oder die Methylgruppe bedeutet.

9. Zusammensetzung nach Anspruch 7 oder 8, **dadurch gekennzeichnet, dass** t=0 und Z, Z' und Z3 -CH2CH2- bedeuten, wobei mindestens ein Wert y oder y' von 0 verschieden ist.

10. Zusammensetzung nach einem der vorhergehenden Ansprüche, **dadurch gekennzeichnet, dass** die Polymere mit Aminoplastether-Grundgerüst unter den folgenden Produkten ausgewählt sind:
PEG-180/Octoxynol-40/TMMG Copolymer,
PEG-180/Laureth-50/TMMG Copolymer,
Polyether-1,
Tetrakis-methoxymethylglycoluril/hydrophob modifizierter Polyether.

11. Zusammensetzung nach einem der Ansprüche 1 bis 10, **dadurch gekennzeichnet, dass** die Polymere mit Aminoplastether-Grundgerüst in einer Menge verwendet werden, die im Bereich von 0,05 bis 3 Gew.-% des Gesamtgewichts der Zusammensetzung liegen kann.

12. Zusammensetzung nach einem der Ansprüche 1 bis 11, **dadurch gekennzeichnet, dass** die Polymere mit Aminoplastether-Grundgerüst in einer Menge verwendet werden, die im Bereich von 0,3 bis 2 Gew.-% des Gesamtgewichts der Zusammensetzung liegen kann.

13. Zusammensetzung nach einem der vorhergehenden Ansprüche, **dadurch gekennzeichnet, dass** sie ferner mindestens ein fixierendes Polymer enthält.

14. Zusammensetzung nach Anspruch 12, **dadurch gekennzeichnet, dass** die relative Konzentration des fixierenden Polymers oder der fixierenden Polymere im Bereich von 0,05 bis 20 Gew.-% und vorzugsweise 0,5 bis 10 Gew.-% liegt.

15. Zusammensetzung nach den Ansprüchen 13 und 14, **dadurch gekennzeichnet, dass** das fixierende Polymer ein anionisches Polymer ist, das ausgewählt ist unter:
- Polymeren, die Carboxyeinheiten enthalten, die von ungesättigten Mono- oder Dicarbonsäuremonomeren der folgenden Formel abgeleitet sind: 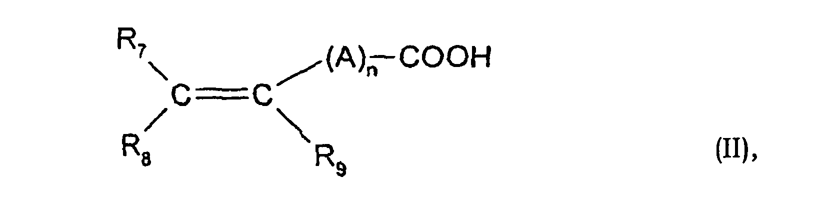 wobei n Null oder eine ganze Zahl von 1 bis 10 bedeutet, A eine Methylengruppe ist, die gegebenenfalls an das Kohlenstoffatom der ungesättigten Gruppe oder, wenn n größer 1 ist, an die benachbarte Methylengruppe über ein Heteroatom, wie Sauerstoff oder Schwefel, gebunden ist, R₇ Wasserstoff, Phenyl oder Benzyl bedeutet, R₈ Wasserstoff, eine niedere Alkylgruppe oder Carboxy ist und R₉ Wasserstoff, eine niedere Alkylgruppe, -CH₂-COOH, Phenyl oder Benzyl bedeutet;
- Polymeren, die Sulfonsäureeinheiten enthalten, wie Vinylsulfonsäureeinheiten, Styrolsulfonsäureeinheiten und Acrylamidoalkylsulfonsäureeinheiten.

16. Zusammensetzung nach den Ansprüchen 13 und 14, **dadurch gekennzeichnet, dass** das fixierende Polymer ein amphoteres Polymer ist, das unter den Polymeren ausgewählt ist, die Einheiten aufweisen, die abgeleitet sind von:
a. mindestens einem Monomer, das unter den Acrylamiden oder Methacrylamiden ausgewählt ist, die am Stickstoffatom mit einer Alkylgruppe substituiert sind,
b. mindestens einem sauren Comonomer, das eine oder mehrere reaktive Carboxygruppen aufweist,
c. mindestens einem basischen Comonomer, wie den Estern von Acrylsäure und Methacrylsäure mit primären, sekundären, tertiären und quartären Aminogruppen als Substituenten und dem Quaternisierungsprodukt von Dimethylaminoethylmethacrylat mit Dimethyl- oder Diethylsulfat.

17. Zusammensetzung nach den Ansprüchen 13 und 14, **dadurch gekennzeichnet, dass** das fixierende Polymer ein nichtionisches Polymer ist, das ausgewählt ist unter:
- Polyalkyloxazolinen;
- Homopolymeren von Vinylacetat;
- Copolymeren von Vinylacetat und Acrylestern;
- Copolymeren von Vinylacetat und Ethylen;
- Copolymeren von Vinylacetat und Maleinsäureestern;
- Copolymeren von Polyethylen und Maleinsäureanhydrid;
- Homopolymeren von Alkylacrylaten und Homopolymeren von Alkylmethacrylaten;
- Copolymeren von Acrylestern, wie beispielsweise Copolymeren von Alkylacrylaten und Alkylmethacrylaten;
- Copolymeren von Acrylnitril und einem nichtionischen Monomer, das beispielsweise unter Butadien und Alkyl(meth)-acrylaten ausgewählt ist;
- Copolymeren von Alkylacrylat und Urethan;
- Polyamiden; und
- nichtionischen Guargummen, die gegebenenfalls chemisch modifiziert sind.

18. Zusammensetzung nach den Ansprüchen 13 und 14, **dadurch gekennzeichnet, dass** das fixierende Polymer ein kationisches Polymer ist, das ausgewählt ist unter:
- Copolymeren von Acrylamid und mit Dimethylsulfat quaternisiertem Dimethylaminoethylmethacrylat;
- Copolymeren von Acrylamid und Methacryloyloxyethyltrimethylammoniummethosulfat;
- dem Copolymer von Acrylamid und Methacryloyloxyethyltrimethylammoniumchlorid;
- quaternisierten oder nicht quaternisierten Copolymeren Vinylpyrrolidon/Dialkylaminoalkylacrylat oder -methacrylat;
- Dimethylaminoethylmethacrylat/Vinylcaprolactam/Vinylpyrrolidon-Terpolymeren;
- dem Vinylpyrrolidon/Dimethylaminopropylmethacrylamid-Copolymer, das quaternisiert ist;
- quaternisierten Polysacchariden, wie Guargummen, die kationische Trialkylammoniumgruppen enthalten;
- quartären Copolymeren von Vinylpyrrolidon und Vinylimidazol;
- Chitosanen oder deren Salzen; und
- kationischen Cellulosederivaten.

19. Zusammensetzung nach den Ansprüchen 13 und 14, **dadurch gekennzeichnet, dass** das fixierende Polymer ein funktionalisiertes oder nicht funktionalisiertes, siliconiertes oder nicht siliconiertes Polyurethan ist.

20. Zusammensetzung nach den Ansprüchen 13 und 14, **dadurch gekennzeichnet, dass** das fixierende Polymer ein Polymer vom Typ eines gepfropften Siliocnpolymers ist, das einen Polysiloxanbereich und einen Bereich enthält, der aus einer nicht siliconhaltigen organischen Kette besteht, wobei einer der beiden Bereiche die Hauptkette des Polymers bildet und der andere auf die Hauptkette gepfropft ist.

21. Zusammensetzung nach einem der vorhergehenden Ansprüche, **dadurch gekennzeichnet, dass** sie ferner ein Konditioniermittel enthält, das aus der Gruppe der Poly-α-olefine, fluorierten Öle, pflanzlichen Öle, natürlichen Wachse, fluorierten Wachse, fluorierten Gummis, Fettsäureester, Organosiloxane, Amidverbindungen, die mindestens eine Fettkette enthalten, und Ceramide ausgewählt ist, wobei diese Stoffe in Form von Gemischen vorliegen können.

22. Zusammensetzung nach einem der vorhergehenden Ansprüche, **dadurch gekennzeichnet, dass** sie mindestens einen Zusatzstoff enthält, der unter den nichtionischen, anionischen, kationischen, amphoteren oder zwitterionischen Tensiden, Parfums, Filtern, Konservierungsmitteln, Proteinen, Vitaminen, nichtionischen, anionischen, kationischen, amphoteren oder zwitterionischen Polymeren, bei denen es sich nicht um erfindungsgemäße Polymere handelt, mineralischen, pflanzlichen oder synthetischen Ölen, allen sonstigen gewöhnlich in Kosmetikzusammensetzungen verwendeten Zusatzstoffen, Wirkstoffen gegen Schuppen, Wirkstoffen gegen Haarausfall, Elektrolyten, Farbstoffen, Pigmenten, feuchtigkeitsspendenden Mitteln, wie Glycerin und anderen Polyolen, und Reduktionsmitteln ausgewählt sind.

23. Kosmetisches Verfahren für die Haarbehandlung, **dadurch gekennzeichnet, dass** eine Zusammensetzung nach einem der vorhergehenden Ansprüche auf das Haar aufgetragen wird.
